# EUROPÄISCHE PATENTANMELDUNG

(11) **EP 0 922 462 A2**
(43) Veröffentlichungstag der Anmeldung: **16.06.1999**
(21) Anmeldenummer: 98122620.2
(22) Anmeldetag: 27.11.1998
(51) Int. Cl.: A61K 47/32, A61K 9/16, A61K 9/20, A61K 9/24

(54) **Verfahren zur Herstellung von festen Dosierungsformen**

(30) Priorität: 01.12.1997 DE 19753300
(71) Anmelder: BASF AKTIENGESELLSCHAFT, 67056 Ludwigshafen (DE)
(72) Erfinder: Kothrade, Stephan, 67117 Limburgerhof (DE); Berndl, Gunther, 67273 Herxheim (DE); Ernst, Andreas, 67551 Worms (DE); Sanner, Axel, 67227 Frankenthal (DE)
(74) Vertreter: Kinzebach, Werner, Dr.

(57) **Zusammenfassung**

Die Erfindung betrifft ein Verfahren zur Herstellung von festen Dosierungsformen durch Vermischen von mindestens einem polymeren Bindemittel, mindestens einem Wirkstoff und gegebenenfalls üblichen Additiven unter Bildung eines plastischen Gemisches, wobei man als polymeres Bindemittel ein Homo- oder Copolymer des N-Vinylcaprolactams verwendet. Das erfindungsgemäße Verfahren ermöglicht die Herstellung von festen Dosierungsformen mit rascher Freisetzung des Wirkstoffes auf einfache und kostengünstige Weise.

## Beschreibung

Die Erfindung betrifft ein Verfahren zur Herstellung von festen Dosierungsformen durch Vermischen von mindestens einem polymeren Bindemittel, mindestens einem Wirkstoff und gegebenenfalls üblichen Additiven unter Bildung eines plastischen Gemisches und Formgebung des Gemisches. Insbesondere betrifft die Erfindung ein Verfahren zur Herstellung von festen pharmazeutischen Dosierungsformen.

Die klassischen Verfahren zur Herstellung fester pharmazeutischer Formen, insbesondere Tabletten, werden diskontinuierlich durchgeführt und umfassen mehrere Stufen. Pharmazeutische Granulate stellen hierbei ein wichtiges Zwischenprodukt dar. So ist z. B. Bauer, Frömmig und Führer, Pharmazeutische Technologie, Georg-Thieme-Verlag, S. 292ff., zu entnehmen, dass man Arzneiformen über Trockengranulierung aus der Schmelze gewinnen kann. Es wird beschrieben, dass Schmelzerstarrungsgranulate entweder durch Schmelzen und Schockerstarren, durch Ausgießen und Zerkleinern oder durch Sprüherstarren in Sprühtürmen hergestellt werden können. Ein Problem bei diesem Verfahren ist die für die Herstellung von Arzneimitteln erforderliche exakte Formgebung. Es werden unregelmäßige Partikel oder Bruchstücke erzeugt, so dass die erzielte Form in keiner Weise den üblichen Arzneiformen entspricht und Granulate deshalb als eigenständige Arzneiform nur eine geringe Bedeutung besitzen. Die Herstellung der gewünschten festen Arzneiformen erfordert den Einsatz weiterer Verfahrensschritte, wie z. B. die Komprimierung mit Hilfe von Tablettiermaschinen. Dies ist zeit- und kostenintensiv.

Seit einiger Zeit ist ein wesentlich einfacheres kontinuierliches Verfahren zur Herstellung fester pharmazeutischer Formen bekannt, bei dem man eine wirkstoffhaltige, lösungsmittelfreie Schmelze aus einem polymeren, wirkstoffhaltigen Bindemittel extrudiert und den extrudierten Strang zu der gewünschten Arzneiform formt, beispielsweise in einem Kalander mit Formwalzen, siehe EP-A-240 904, EP-A-240 906, EP-A-337 256 und EP-A-358 105 (Schmelzextrusion). Damit kann eine gezielte Formgebung erreicht werden. Als polymeres Bindemittel werden insbesondere Polymere des N-Vinylpyrrolidon oder Copolymerisate davon, z. B. mit Vinylacetat, eingesetzt.

Homo- und Copolymere des N-Vinylcaprolactams wurden bereits in herkömmlichen pharmazeutischen Dosierungsformen verwendet. So beschreibt die US 3,900,559 eine Dosierungsform für die anhaltende Freisetzung von Methanthelin aus einer Matrix, die ein wasserunlösliches, gelbildendes, hydrophiles Terpolymer mit N-Vinyllactam as einem Bestandteil umfasst. Die US 4,987,182 beschreibt die Herstellung eines Gemisches aus Polyvinylbutyral und einem Polyvinyllactam, das als Wirkstoffträger verwendet wird. Die WO 96/17579 beschreibt ein Bindemittel für Tabletten, das ein Homo- oder Copolymer eines N-Vinyllactams und eine Polysäure enthält. Die EP 756 820 A beschreibt iodhaltige Zubereitungen mit verbesserter Stabilität, die Polyvinylcaprolactam in der Bindemittelkomponente enthalten können. In der DE 44 34 986 A ist die Herstellung von Polyvinylcaprolactam beschrieben. Dieses wird zur Herstellung von langsam freisetzenden Wirkstoffzubereitungen eingesetzt. Die DE 43 27514 A offenbart ein Copolymer aus einem wasserunlöslichen Monomer und einem wasserlöslichen Monomer, bei dem es sich um N-Vinylcaprolactam handeln kann. Das Copolymer ist als Träger für Wirkstoffe brauchbar und ermöglicht eine leichtere Einarbeitung der Komponenten und kontrollierte Freisetzung.

Die DE 44 22 881 beschreibt die Verwendung von Copolymeren des N-Vinylcaprolactams mit hydrophoben Comonomeren in Formulierungen von schwer wasserlöslichen Wirkstoffen. Die Formulierungen liegen in Form von Lösungen oder Dispersionen vor und besitzen verbesserte Stabilität und Wirksamkeit.

Die DE 35 17 080 beschreibt eine Komponente für therapeutische Wirkstoffabgabesysteme auf Basis von Polieren mit Kautschukeigenschaften, bei denen es sich um Homo- oder Copolymere von N-Vinyllactamen handeln kann. Diese Komponenten werden in der Wirkstoffreservoirschicht für therapeutische Wirkstoffabgabesysteme, beispielsweise medizinische Pflaster, verwendet.

Dosierungsformen auf Basis derartiger Polymere haben den Nachteil, dass sie den Wirkstoff für viele Anwendungsfälle zu langsam freisetzen. Es ist daher nicht möglich, rasch freisetzende Dosierungsformen, sogenannte "instant release"-Formulierungen, herzustellen, ohne zusätzliche Maßnahmen zu ergreifen, wie beispielsweise das Zerkleinern der Dosierungsform, um eine große Oberfläche zu erzeugen.

Der vorliegenden Erfindung liegt daher die Aufgabe zu Grunde, Dosierungsformen zur Verfügung zu stellen, die sich durch Schmelzextrusion herstellen lassen und in der Lage sind, den Wirkstoff rasch freizusetzen.

Überraschenderweise wurde nun gefunden, dass diese Aufgabe gelöst wird, wenn man als polymeres Bindemittel ein N-vinylcaprolactamhaltiges Polymerisat verwendet.

Gegenstand der vorliegenden Erfindung ist daher ein Verfahren zur Herstellung von festen Dosierungsformen durch Vermischen von mindestens einem polymeren Bindemittel, mindestens einem Wirkstoff und gegebenenfalls üblichen Additiven unter Bildung eines plastischen Gemisches und Formgebung, wobei das Verfahren dadurch gekennzeichnet ist, dass man als polymeres Bindemittel ein Homo- oder Copolymer von N-Vinylcaprolactam verwendet.

Das erfindungsgemäße Verfahren ermöglicht die Herstellung von festen Dosierungsformen mit rascher Freisetzung des Wirkstoffs ("instant release") auf einfache und kostengünstige Weise. Die rasche Freisetzung des Wirkstoffes ist überraschend, weil N-Vinylcaprolactampolymere gemäß Stand der Technik den Wirkstoff nur langsam freisetzen und weil aus plastischen Gemischen der Komponenten sehr kompakte, nicht poröse Dosierungsformen mit geringer Oberfläche erhalten werden, was eine langsame Wirkstofffreisetzung erwarten lassen würde.

Unter Dosierungsformen sind hier alle Formen zu verstehen, die zur Verwendung als Arzneimittel, Pflanzenbehandlungsmittel, Futtermittel und Nahrungsmittel und zur Abgabe von Riechstoffen und Parfümölen geeignet sind. Dazu gehören beispielsweise Tabletten jeglicher Form, Pellets, Granulate, aber auch größere Formen, wie Würfel, Blöcke (Quader) oder zylindrische Formen, die sich insbesondere als Futter- oder Nahrungsmittel verwenden lassen.

Die erfindungsgemäß erhältlichen Dosierungsformen umfassen im Allgemeinen:
a) 0,1 bis 90 Gew.-%, insbesondere 0,1 bis 60 Gew.-% (bezogen auf das Gesamtgewicht der Dosierungsform) eines Wirkstoffes,
b) 10 bis 99,9 Gew.-%, insbesondere 40 bis 99,9 Gew.-% eines polymeren Bindemittels und
c) gegebenenfalls Additive.

Als polymeres Bindemittel kann ein Homopolymer oder ein Copolymer von N-Vinylcaprolactam eingesetzt werden. Das Copolymer enthält mindestens 1 Gew.-%, vorzugsweise mindestens 10 Gew.-%, besonders bevorzugt mindestens 25 Gew.-% und insbesondere mindestens 50 Gew.-% N-Vinylcaprolactameinheiten.

Als Copolymer enthält das polymere Bindemittel mindestens ein weiteres copolymerisierbares Monomer in einer Menge von 0,5 bis 99 Gew.-%, vorzugsweise 0,5 bis 90 Gew.-%, besonders bevorzugt 0,5 bis 75 Gew.-% und insbesondere 0,5 bis 50 Gew.-% einpolymerisiert. Geeignete Comonomere sind insbesondere monoethylenisch ungesättigte Carbonsäuren mit 3 bis 30, insbesondere 3 bis 8 Kohlenstoffatomen, wie Acrylsäure, Methacrylsäure, Dimethylacrylsäure, Ethacrylsäure, Maleinsäure, Citraconsäure, Methylenmalonsäure, Allylessigsäure, Vinylessigsäure, Crotonsäure, Fumarsäure, Mesaconsäure und Itaconsäure sowie die Halbester der erwähnten Dicarbonsäuren mit C₁- bis C₂₄-Alkanolen. Bevorzugt sind Acrylsäure, Methacrylsäure, Maleinsäure oder Mischungen der genannten Carbonsäuren. Die monoethylenisch ungesättigten Carbonsäuren können in Form der freien Säure und, soweit vorhanden, der Anhydride oder in partiell oder vollständig neutralisierter Form eingesetzt werden. Zur Neutralisation verwendet man vorzugsweise Alkalimetall- oder Erdalkalimetallbasen, Ammoniak oder Amine, z. B. Natriumhydroxid, Kaliumhydroxid, Natriumcarbonat, Kaliumcarbonat, Natriumhydrogencarbonat, Magnesiumoxid, Calciumhydroxid, Calciumoxid, gasförmiges oder wässriges Ammoniak, Triethylamin, Ethanolamin, Diethanolamin, Morpholin, Diethylentriamin, oder Tetraethylenpentamin.

Weiterhin eignen sich als Comonomere beispielsweise die Ester der oben angegebenen Carbonsäuren mit C₁- bis C₂₄-Alkanolen, C₂- bis C₄-Diolen, Mono- und Di-C₁ bis C₄-alkylamino-C₂- bis C₄-alkanolen sowie die Amide, Mono- und Di-C₁- bis C₄-alkylamide und Nitrile dieser Carbonsäuren, z. B. Acrylsäuremethylester, Acrylsäureethylester, Methacrylsäuremethylester, Methacrylsäureethylester, Hydroxy-ethylacrylat, Hydroxypropylacrylat, Hydroxybutylacrylat, Hydroxyethylmethacrylat, Hydroxypropylmethacrylat, Hydroxyisobutylacrylat, Hydroxyisobutylmethacrylat, Maleinsäuremonomethylester, Maleinsäuredimethylester, Maleinsäuremonoethylester, Maleinsäurediethylester, 2-Ethylhexylacrylat, 2-Ethylhexylmethacrylat, Stearylacrylat, Stearylmethacrylat, Behenylacrylat, Behenylmethacrylat, Octylacrylat, Octylmethacrylat, Acrylamid, Methacrylamid, N,N-Dimethylacrylamid, N-tert.-Butylacrylamid, Acrylnitril, Methacrylnitril, Dimethylaminoethylacrylat, Diethylaminoethylacrylat, Diethylaminoethylmethacrylat sowie die Salze der zuletzt genannten Monomeren mit Carbonsäuren oder Mineralsäuren oder die quaternisierten Produkte.

Außerdem eignen sich als copolymerisierbare Monomere Acrylamidoglykolsäure, Vinylsulfonsäure, Allylsulfonsäure, Methallylsulfonsäure, Styrolsulfonsäure, Acrylsäure-(3-sulfopropyl)ester, Methacrylsäure-(3-sulfopropyl)ester und Acrylamidomethylpropansulfonsäure sowie Phosphonsäuregruppen enthaltende Monomere wie Vinylphosphonsäure, Allylphosphonsäure und Acrylamidomethylpropanphosphonsäure. Weitere geeignete copolymerisierbare Monomere sind N-Vinylpyrrolidon, N-Vinylimidazol, N-Vinyl-2-methylimidazol, N-Vinyl-4-methylimidazol, Diallylammoniumchlorid und Vinylester, wie Vinylacetat und Vinylpropionat, sowie Vinylaromaten, wie Styrol. Es können selbstverständlich auch Mischungen der genannten Monomere eingesetzt werden.

Die Herstellung der Homo- und Copolymerisate erfolgt nach bekannten Verfahren, z. B. der Lösungs-, Fällungs-, Suspensions- oder umgekehrten Suspensionspolymerisation, oder der Emulsions- bzw. umgekehrten Emulsionspolymerisation unter Verwendung von Verbindungen, die unter Polymerisationsbedingungen Radikale bilden.

Die Polymerisationstemperaturen liegen üblicherweise in dem Bereich von 30 bis 200 °C, vorzugsweise 40 bis 110 °C. Geeignete Initiatoren sind beispielsweise Azo- und Peroxyverbindungen sowie die üblichen Redoxinitiatorsysteme, wie Kombinationen aus Wasserstoffperoxid und reduzierend wirkende Verbindungen, z. B. Natriumsulfit, Natriumbisulfit, Natriumformaldehydsulfoxilat und Hydrazin.

Die Copolymeren besitzen K-Werte von mindestens 7, vorzugsweise 10 bis 100, besonders bevorzugt 10 bis 50. Die K-Werte werden bestimmt nach H. Fikentscher, Cellulose-Chemie, Band 13, (1932) 58-64 und 71-74, in wässriger Lösung oder in einem organischen Lösungsmittel bei 25 °C, bei Konzentrationen, die je nach K-Wert-Bereich zwischen 0,1 % und 5 % liegen.

Neben den oben beschriebenen polymeren Bindemitteln können, insbesondere bis zu 30 Gew.-%, weitere Bindemittel, bezogen auf das Gesamtgewicht des Bindemittels, eingesetzt werden. Geeignet sind Polymere, Copolymere, Cellulosederivate, Stärke und Stärkederivate, beispielsweise:

Polyvinylpyrrolidon (PVP), Copolymerisate von N-Vinylpyrrolidon (NVP) und Vinylacetat oder Vinylpropionat, Copolymerisate von Vinylacetat und Crotonsäure, teilverseiftes Polyvinylacetat, Polyvinylalkohol, Polyhydroxyalkylacrylate, Polyhydroxyalkylmethacrylate, Polyacrylate und Polymethacrylate (Eudragit-Typen), Copolymerisate von Methylmethacrylat und Acrylsäure, Polyacrylamide, Polyethylenglykole, Polyvinylformamid (gegebenenfalls partiell oder vollständig hydrolysiert), Celluloseester, Celluloseether, insbesondere Methylcellulose und Ethylcellulose, Hydroxyalkylcellulosen, insbesondere Hydroxypropylcellulose, Hydroxyalkyl-Alkylcellulosen, insbesondere Hydroxypropyl-Ethylcellulose, Cellulosephthalate, insbesondere Celluloseacetatphthalat und Hydroxypropylmethylcellulosephthalat, und Mannane, insbesondere Galactomannane. Davon sind Polyvinylpyrrolidon, Copolymerisate von N-Vinylpyrrolidon und Vinylestern, Polyhydroxyalkylacrylate, Polyhydroxyalkylmethacrylate, Polyacrylate, Polymethacrylate, Alkylcellulosen und Hydroxyalkylcellulosen besonders bevorzugt.

Das polymere Bindemittel muß in der Gesamtmischung aller Komponenten im Bereich von 50 bis 180 °C, vorzugsweise 60 bis 130 °C erweichen oder schmelzen, wobei sich ein plastisches Gemisch bildet. Die Glasübergangstemperatur der Mischung muss daher unter 180 °C, vorzugsweise unter 130 °C liegen. Erforderlichenfalls wird sie durch übliche, pharmakologisch akzeptable weichmachende Hilfsstoffe herabgesetzt. Die Menge an Weichmacher beträgt höchstens 30 Gew.-%, bezogen auf das Gesamtgewicht von Bindemittel und Weichmacher, damit lagerstabile Arzneiformen gebildet werden, die keinen kalten Fluss zeigen. Vorzugsweise aber enthält das Gemisch keinen Weichmacher.

Beispiele für derartige Weichmacher sind:
langkettige Alkohole, Ethylenglykol, Propylenglykol, Glycerin, Trimethylolpropan, Triethylenglykol, Butandiole, Pentanole, wie Pentaerythrit, Hexanole, Polyethylenglykole, Polypropylenglykole, Polyethylenpropylenglykole, Silicone, aromatische Carbonsäureester (z. B. Dialkylphthalate, Trimellithsäureester, Benzoesäureester, Terephthalsäureester) oder aliphatische Dicarbonsäureester (z. B. Dialkyladipate, Sebacinsäureester, Azelainsäureester, Zitronen- und Weinsäureester), Fettsäureester, wie Glycerinmono-, Glycerindi- oder Glycerintriacetat oder Natriumdiethylsulfosuccinat. Die Konzentration an Weichmacher beträgt im Allgemeinen 0,5 bis 15, vorzugsweise 0,5 bis 5 Gew.-%, bezogen auf das Gesamtgewicht des Gemisches.

Übliche galenische Hilfsstoffe, deren Gesamtmenge bis zu 100 Gew.-%, bezogen auf das Polymerisat, betragen kann, sind z. B. Streckmittel bzw. Füllstoffe, wie Silikate oder Kieselerde, Magnesiumoxid, Aluminiumoxid, Titanoxid, Stearinsäure oder deren Salze, z. B. das Magnesium- oder Calciumsalz, Methylcellulose, Natrium-Carboxymethylcellulose, Talkum, Saccharose, Lactose, Getreide- oder Maisstärke, Kartoffelmehl, Polyvinylalkohol, insbesondere in einer Konzentration von 0,02 bis 50, vorzugsweise 0,20 bis 20 Gew.-%, bezogen auf das Gesamtgewicht des Gemisches. Schmiermittel, wie Aluminium- und Calciumstearat, Talkum und Silicone, in einer Konzentration von 0,1 bis 5, vorzugsweise 0,1 bis 3 Gew.-%, bezogen auf das Gesamtgewicht des Gemisches.

Fließmittel, wie tierische oder pflanzliche Fette, insbesondere in hydrierter Form und solche, die bei Raumtemperatur fest sind. Diese Fette haben vorzugsweise einen Schmelzpunkt von 50°C oder höher. Bevorzugt sind Triglyceride der C₁₂-, C₁₄-, C₁₆- und C₁₈-Fettsäuren. Auch Wachse, wie Carnaubawachs, sind brauchbar. Diese Fette und Wachse können vorteilhaft alleine oder zusammen mit Mono- und/oder Diglyceriden oder Phosphatiden, insbesondere Lecithin, zugemischt werden. Die Mono- und Diglyceride stammen vorzugsweise von den oben erwähnten Fettsäuretypen ab. Die Gesamtmenge an Fetten, Wachsen, Mono-, Diglyceriden und/oder Lecithinen beträgt 0,1 bis 30, vorzugsweise 0,1 bis 5 Gew.-%, bezogen auf das Gesamtgewicht der Masse für die jeweilige Schicht;

Farbstoffe, wie Azofarbstoffe, organische oder anorganische Pigmente oder Farbstoffe natürlicher Herkunft, wobei anorganische Pigmente in einer Konzentration von 0,001 bis 10, vorzugsweise 0,5 bis 3 Gew.-%, bezogen auf das Gesamtgewicht des Gemisches bevorzugt sind;

Stabilisatoren, wie Antioxidanzien, Lichtstabilisatoren, Hydroperoxid-Vernichter, Radikalfänger, Stabilisatoren gegen mikrobiellen Befall.

Ferner können Netz-, Konservierungs-, Spreng-, Adsorptions-, Formentrenn- und Treibmittel zugesetzt werden (vgl. z. B. H. Sucker et al., Pharmazeutische Technologie, Thieme-Verlag, Stuttgart 1978).

Unter Hilfsstoffen im Sinne der Erfindung sind auch Substanzen zur Herstellung einer festen Lösung des Wirkstoffs zu verstehen. Diese Hilfsstoffe sind beispielsweise Pentaerythrit und Pentaerythrit-tetraacetat, Polymere wie z. B. Polyethylen- bzw. Polypropylenoxide und deren Blockcopolymere (Poloxamere), Phosphatide wie Lecithin, Homo- und Copolymere des Vinylpyrrolidons, Tenside wie Polyoxyethylen-40-stearat sowie Zitronen- und Bernsteinsäure, Gallensäuren, Sterine und andere, wie z. B. bei J. L. Ford, Pharm. Acta Helv. 61, 69-88 (1986) angegeben.

Als Hilfsstoffe gelten auch Zusätze von Basen und Säuren zur Steuerung der Löslichkeit eines Wirkstoffes (siehe beispielsweise K. Thoma et al., Pharm. Ind. 51, 98-101 (1989)).

Einzige Voraussetzung für die Eignung von Hilfsstoffen ist eine ausreichende Temperaturstabilität.

Unter Wirkstoffen im Sinne der Erfindung sind alle Stoffe mit einer physiologischen Wirkung zu verstehen, sofern sie sich unter den Verarbeitungsbedingungen nicht zersetzen. Es handelt sich insbesondere um pharmazeutische Wirkstoffe (für Mensch und Tier), Wirkstoffe für die Pflanzenbehandlung, Insektizide, Futter- und Nahrungsmittelwirkstoffe, Riechstoffe und Parfümöle. Die wirkstoffmenge pro Dosiseinheit und die Konzentration können je nach Wirksamkeit und Freisetzungsgeschwindigkeit in weiten Grenzen variieren. Die einzige Bedingung ist, dass sie zur Erzielung der gewünschten Wirkung ausreichen. So kann die Wirkstoffkonzentration im Bereich von 0,1 bis 95, vorzugsweise von 20 bis 80, insbesondere 30 bis 70 Gew. -% liegen. Auch Wirkstoff-Kombinationen können eingesetzt werden. Wirkstoffe im Sinne der Erfindung sind auch Vitamine und Mineralstoffe. Zu den Vitaminen gehören die Vitamine der A-Gruppe, der B-Gruppe, worunter neben B₁, B₂, B₆ und B₁₂ sowie Nicotinsäure und Nicotinamid auch Verbindungen mit Vitamin B-Eigenschaften verstanden werden, wie z. B. Adenin, Cholin, Pantothensäure, Biotin, Adenylsäure, Folsäure, Orotsäure, Pangamsäure, Carnitin, p-Aminobenzoesäure, myo-Inosit und Liponsäure sowie Vitamin C, Vitamine der D-Gruppe, E-Gruppe, F-Gruppe, H-Gruppe, I- und J-Gruppe, K-Gruppe und P-Gruppe. Zu Wirkstoffen im Sinne der Erfindung gehören auch Peptidtherapeutika. Zu Pflanzenbehandlungsmitteln zählen z. B. Vinclozolin, Epoxiconazol und Quinmerac.

Das erfindungsgemäße Verfahren ist beispielsweise zur Verarbeitung folgender Wirkstoffe geeignet:

Acebutolol, Acetylcystein, Acetylsalicylsäure, Aciclovir, Alprazolam, Alfacalcidol, Allantoin, Allopurinol, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium-Hydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefalexin, Cefadroxil, Cefazolin, Cefixim, Cefotaxim, Ceftazidim, Ceftriaxon, Cefuroxim, Selegilin, Chloramphenicol, Chlorhexidin, Chlor-pheniramin, Chlortalidon, Cholin, Cyclosporin, Cilastatin, Cimetidin, Ciprofloxacin, Cisapride, Cisplatin, Clarithromycin, Clävulansäure, Clomipramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, Doxycyclin, Enalapril, Ephedrin, Epinephrin, Ergocalciferol, Ergotamin, Erythromycin, Estradiol, Ethinylestradiol, Etoposid, Eucalyptus Globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Furosemid, Gallopamil, Gemfibrozil, Gentamicin, Gingko Biloba, Glibenclamid, Glipizid, Glozapin, Glycyrrhiza glabra, Griseofulvin, Guaifenesin, Haloperidol, Heparin, Hyaluronsäure, Hydrochlorothiazid, Hydrocodon, Hydrocortison, Hydromorphon, Ipratropium-Hydroxid, Ibuprofen, Imipenem, Indomethacin, Iohexol, Iopamidol, Isosorbid-Dinitrat, Isosorbid-Mononitrat, Isotretinoin, Ketotifen, Ketoconazol, Ketoprofen, Ketorolac, Labetalol, Lactulose, Lecithin, Levocarnitin, Levodopa, Levoglutamid, Levonorgestrel, Levothyroxin, Lidocain, Lipase, Imipramin, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -Kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Phenobarbital, Pentoxifyllin, Phenoxymethylpenicillin, Phenylephrin, Phenylpropanolamin, Phenytoin, Piroxicam, Polymyxin B, Povidon-Iod, Pravastatin, Prazepam, Prazosin, Prednisolon, Prednison, Bromocriptin, Propafenon, Propranolol, Proxyphyllin, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Simvastatin, Somatropin, Sotalol, Spironolacton, Sucralfat, Sulbactam, Sulfamethoxazol, Sulfasalazin, Sulpirid, Tamoxifen, Tegafur, Teprenon, Terazosin, Terbutalin, Terfenadin, Tetracyclin, Theophyllin, Thiamin, Ticlopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin E, Folinsäure, Zidovudin.

Bevorzugte Wirkstoffe sind Ibuprofen (als Racemat, Enantiomer oder angereichertes Enantiomer), Ketoprofen, Flurbiprofen, Acetylsalicylsäure, Verapamil, Paracetamol, Nifedipin oder Captopril.

Zur Herstellung der festen Dosierungsformen wird ein plastisches Gemisch der Komponenten (Schmelze) bereitgestellt, das anschließend einem Formgebungsschnitt unterzogen wird. Das Vermischen der Komponenten und die Bildung der Schmelze können auf unterschiedliche Weise erfolgen. Das Vermischen kann vor, während und/oder nach der Bildung der Schmelze erfolgen. Beispielsweise können die Komponenten zuerst vermischt und dann aufgeschmolzen oder gleichzeitig vermischt und aufgeschmolzen werden. Häufig erfolgt noch eine Homogenisierung des plastischen Gemisches, um eine hochdisperse Verteilung des Wirkstoffes zu erhalten.

Insbesondere bei Verwendung von empfindlichen Wirkstoffen hat es sich aber als bevorzugt erwiesen, zuerst das polymere Bindemittel, gegebenenfalls zusammen mit üblichen pharmazeutischen Additiven, aufzuschmelzen und vorzuvermischen und dann den (die) empfindlichen Wirkstoff(e) in "Intensivmischern" in plastischer Phase bei sehr kleinen Verweilzeiten einzumischen (Homogenisieren). Der (die) Wirkstoff(e) kann (können) dabei in fester Form oder als Lösung oder Dispersion eingesetzt werden.

Im Allgemeinen werden die Komponenten als solche in das Herstellungsverfahren eingesetzt. Sie können jedoch auch in flüssiger Form, d. h. als Lösung, Suspension oder Dispersion zur Anwendung kommen.

Als Lösungsmittel für die flüssige Form der Komponenten kommt in erster Linie Wasser oder ein mit Wasser mischbares, organisches Lösungsmittel oder ein Gemisch davon mit Wasser in Betracht. Brauchbare Lösungsmittel sind aber auch mit Wasser nicht mischbare oder mischbare, organische Lösungsmittel. Geeignete, mit Wasser mischbare Lösungsmittel sind insbesondere C₁-C₄-Alkanole, wie Ethanol, Isopropanol oder n-Propanol, Polyole, wie Ethylenglykol, Glycerin und Polyethylenglykole. Geeignete, mit Wasser nicht mischbare Lösungsmittel sind Alkane, wie Pentan oder Hexan, Ester, wie Ethylacetat oder Butylacetat, chlorierte Kohlenwasserstoffe, wie Methylenchlorid und aromatische Kohlenwasserstoffe, wie Toluol und Xylol. Ein weiteres brauchbares Lösungsmittel ist flüssiges CO₂.

Welches Lösungsmittel im Einzelfall verwendet wird, hängt von der aufzunehmenden Komponente und deren Eigenschaften ab. Beispielsweise kommen pharmazeutische Wirkstoffe häufig in Form eines Salzes, das im Allgemeinen wasserlöslich ist, zur Anwendung. Wasserlösliche Wirkstoffe können daher als wässrige Lösung eingesetzt werden oder vorzugsweise in die wässrige Lösung oder Dispersion des Bindemittels aufgenommen werden. Entsprechendes gilt für Wirkstoffe, die in einem der genannten Lösungsmittel löslich sind, wenn die flüssige Form der zur Anwendung kommenden Komponenten auf einem organischen Lösungsmittel basiert.

Gegebenenfalls kann an die Stelle des Aufschmelzens ein Lösen, Suspendieren oder Dispergieren in den oben genannten Lösungsmitteln, falls erwünscht und/oder erforderlich unter Zusatz geeigneter Hilfsstoffe, wie z. B. Emulgatoren, treten. Das Lösungsmittel wird dann im Allgemeinen unter Bildung der Schmelze in einer geeigneten Apparatur, z. B. einem Extruder, entfernt. Im Folgenden soll dies von dem Begriff Vermischen umfasst werden.

Das Aufschmelzen und/oder Vermischen erfolgt in einer für diesen Zweck üblichen Vorrichtung. Besonders geeignet sind Extruder oder gegebenenfalls beheizbare Behälter mit Rührwerk, z. B. Kneter, (wie der unten noch erwähnten Art).

Als Mischapparat sind insbesondere solche Vorrichtungen brauchbar, die in der Kunststofftechnologie zum Mischen eingesetzt werden. Geeignete Vorrichtungen sind beispielsweise beschrieben in "Mischen beim Herstellen und Verarbeiten von Kunststoffen", H. Pahl, VDI-Verlag, 1986. Besonders geeignete Mischapparaturen sind Extruder und dynamische und statische Mischer, sowie Rührkessel, einwellige Rührwerke mit Abstreifvorrichtungen, insbesondere sogenannte Pastenrührwerke, mehrwellige Rührwerke, insbesondere PDSM-Mischer, Feststoffmischer sowie vorzugsweise Misch-Knetreaktoren (z. B. ORP, CRP, AP, DTB der Firma List oder Reactotherm der Firma Krauss-Maffei oder Ko-Kneter der Fa. Buss), Doppelmuldenkneter (Trogmischer) und Stempelkneter (Innenmischer) oder Rotor/Stator-Systeme (z. B. Dispax der Firma IKA).

Bei empfindlichen Wirkstoffen erfolgt vorzugsweise zunächst das Aufschmelzen des polymeren Bindemittels in einem Extruder und anschließend das Zumischen des Wirkstoffs in einem Misch-Knetreaktor. Bei weniger empfindlichen Wirkstoffen kann man dagegen zum intensiven Dispergieren des Wirkstoffs ein Rotor/Stator-System einsetzen.

Das Beschicken der Mischvorrichtung erfolgt je nach deren Konzeption kontinuierlich oder diskontinuierlich in üblicher Weise. Pulverförmige Komponenten können im freien Zulauf, z. B. über eine Differentialdosierwaage eingeführt werden. Plastische Massen können direkt aus einem Extruder eingespeist oder über eine Zahnradpumpe, die insbesondere bei hohen Viskositäten und hohen Drüken von Vorteil ist, zugespeist werden. Flüssige Medien können über ein geeignetes Pumpenaggregat zudosiert werden.

Das durch Vermischen und/oder Aufschmelzen des Bindemittels, des Wirkstoffes und gegebenenfalls des Additivs oder der Additive erhaltene Gemisch ist teigig bis zähflüssig (thermoplastisch) oder flüssig und daher extrudierbar. Die Glasübergangstemperatur des Gemisches liegt unter der Zersetzungstemperatur aller in dem Gemisch enthaltenen Komponenten. Das Bindemittel soll vorzugsweise in physiologischer Umgebung löslich oder quellbar sein.

Die Verfahrensschritte Vermischen und Aufschmelzen können in derselben Apparatur oder in zwei oder mehreren getrennt arbeitenden Vorrichtungen ausgeführt werden. Die Zubereitung einer Vorm)schung kann in einer der oben beschriebenen üblichen Mischvorrichtungen durchgeführt werden. Eine solche Vormischung kann dann direkt, z. B. in einen Extruder, eingespeist und anschließend ggf. unter Zusatz weiterer Komponenten extrudiert werden.

Das erfindungsgemäße Verfahren erlaubt es, als Extruder Einschneckenmaschinen, kämmende Schneckenmaschinen oder auch Mehrwellextruder, insbesondere Zweischnecken-Extruder, gleichsinnig oder gegensinnig drehend und gegebenenfalls mit Knetscheiben ausgerüstet, einzusetzen. Wenn bei der Extrusion ein Lösungsmittel verdampft werden muss, sind die Extruder im Allgemeinen mit einem Verdampfungsteil ausgerüstet. Besonders bevorzugt sind Extruder der ZKS-Baureihe von Werner u. Pfleiderer.

Erfindungsgemäß können auch mehrschichtige pharmazeutische Formen durch Koextrusion hergestellt werden, wobei mehrere Gemische aus den oben beschriebenen Komponenten bei der Extrusion so in einem Werkzeug zusammengeführt werden, dass sich der gewünschte Schichtaufbau der mehrschichtigen pharmazeutischen Form ergibt. Vorzugsweise verwendet man verschiedene Bindemittel für verschiedene Schichten.

Mehrschichtige Arzneiformen umfassen vorzugsweise zwei oder drei Schichten. Sie können in offener oder geschlossener Form vorliegen, insbesondere als offene oder geschlossene Mehrschichttabletten.

Wenigstens eine der Schichten enthält wenigstens einen pharmazeutischen Wirkstoff. Es ist auch möglich, einen weiteren Wirkstoff in eine andere Schicht aufzunehmen. Dies hat den Vorteil, dass zwei miteinander unverträgliche Wirkstoffe verarbeitet werden können oder dass die Freisetzungscharakteristik des Wirkstoffes gesteuert werden kann.

Das Ausformen erfolgt durch Koextrusion, wobei die Gemische aus den einzelnen Extrudern oder anderen Aggregaten in ein gemeinsames Koextrusionswerkzeug geführt und ausgetragen werden. Die Form der Koextrusionswerkzeuge richtet sich nach der gewünschten pharmazeutischen Form. Beispielsweise sind Werkzeuge mit ebenem Austrittsspalt, sogenannte Breitschlitzwerkzeuge, und Werkzeuge mit kreisringspaltförmigem Austrittsquerschnitt geeignet. Die Düsenauslegung erfolgt dabei in Abhängigkeit von dem zur Anwendung kommenden polymeren Bindemittel und der gewünschten pharmazeutischen Form.

Das erhaltene Gemisch ist vorzugsweise lösungsmittelfrei, d.h. es enthält weder Wasser noch ein organisches Lösungsmittel.

Das plastische Gemisch wird in der Regel einer abschließenden Formgebung unterzogen. Dabei kann eine Vielzahl von Formen, je nach Werkzeug und Art der Formung, erzeugt werden. Beispielsweise lässt sich bei Verwendung eines Extruders der extrudierte Strang zwischen einem Band und einer Walze, zwischen zwei Bändern oder zwischen zwei Walzen, wie in der EP-A-358 105 beschrieben, oder durch Kalandrierung in einem Kalander mit zwei Formwalzen, siehe beispielsweise EP-A-240 904, formen. Durch Extrusion und Heiß- oder Kaltabschlag des Stranges können weitere Formen erhalten werden, beispielsweise kleinteilige und gleichmäßig geformte Granulate. Die Heißgranulierung führt in der Regel zu linsenförmigen Dosierungsformen (Tabletten) mit einem Durchmesser von 1 bis 10 mm, während die Kaltgranulierung normalerweise zu zylinderförmigen Produkten mit einem Verhältnis von Länge zu Durchmesser von 1 bis 10 und einem Durchmesser von 0,5 bis 10 mm führt. So können einschichtige, bei Anwendung der Koextrusion aber auch offene oder geschlossene, mehrschichtige Dosierungsformen hergestellt werden, beispielsweise Oblongtabletten, Dragees, Pastillen und Pellets. Die erhaltenen Granulate können anschließend auch zu Pulver gemahlen und in üblicher Weise zu Tabletten verpresst werden. Mikropastillen können durch das Rotoform-Sandvik-Verfahren hergestellt werden. Diese Dosierungsformen können in einem nachgeschalteten Verfahrensschritt nach üblichen Methoden gerundet und/oder mit einem Coating versehen werden. Geeignete Materialien für Filmüberzüge sind z. B. Polyacrylate, wie die Eudragit-Typen, Celluloseester, wie die Hydroxypropylcellulosephthalate, sowie Celluloseether, wie Ethylcellulose, Hydroxypropylmethylcellulose oder Hydroxypropylcellulose.

Im Einzelnen kann es zur Ausbildung von festen Lösungen kommen. Der Begriff "feste Lösungen" ist dem Fachmann geläufig, beispielsweise aus der eingangs zitierten Literatur. In festen Lösungen von Wirkstoffen in Polymeren liegt der Wirkstoff molekulardispers im Polymer vor.

Die folgenden Beispiele sollen das erfindungsgemäße Verfahren veranschaulichen, ohne es jedoch zu beschränken.

### Beispiele

### Beispiel 1

520 g Polyvinylcaprolactam (K-Wert 40; 1%ig in Ethanol) wurden mit 480 g Verapamil-Hydrochlorid unter den nachfolgend angegebenen Bedingungen extrudiert und zu 500 mg-Oblong-Tabletten extrudiert und kalandriert.

| | |
|---|---|
| Schuss 1 | 52 °C |
| Schuss 2 | 85 °C |
| Schuss 3 | 130 °C |
| Schuss 4 | 108 °C |
| Schuss 5 | 92 °C |
| Düse | 85 °C |

Die Freisetzung nach 1 Stunde betrug 100 % (Paddle-Modell nach USP (pH change)).

### Beispiel 2

500 g Copolymer aus 50 Gew. -% Vinylcaprolactam und 50 Gew. -% Vinylpyrrolidon (K-Wert 65; 1%ig in Wasser) wurden mit 480 g Verapamil-Hydrochlorid unter den nachfolgend angegebenen Bedingungen zu 500 mg Oblong-Tabletten extrudiert und granuliert.

| | |
|---|---|
| Schuss 1 | 47 °C |
| Schuss 2 | 86 °C |
| Schuss 3 | 132 °C |
| Schuss 4 | 112 °C |
| Schuss 5 | 101 °C |
| Düse | 100 °C |

Die Freisetzung nach 1 Stunde betrug 67 %, nach 2 Stunden 100 % (Paddle-Modell nach USP (pH change)).

### Beispiel 3

500 g Copolymer aus 50 Gew.-% Vinylcaprolactam und 50 Gew. -% Vinylpyrrolidon (K-Wert 65; 1%ig in Wasser) wurden mit 500 g Vinclozolin unter den nachfolgend angegebenen Bedingungen extrudiert, gekühlt und granuliert.

| | |
|---|---|
| Schuss 1 | 60 °C |
| Schuss 2 | 96 °C |
| Schuss 3 | 140 °C |
| Schuss 4 | 150 °C |
| Schuss 5 | 129 °C |
| Düse | 100 °C |

Es wurde ein transparentes, röntgenamorphes in Wasser dispergierbares Granulat erhalten.

### Beispiel 4

500 g Polyvinylcaprolactam (K-Wert 40; 1%ig in Ethanol) wurden mit 500 g Epoxiconazol unter den nachfolgend angegebenen Bedingungen extrudiert, gekühlt und kalandriert.

| | |
|---|---|
| Schuss 1 | 60 °C |
| Schuss 2 | 90 °C |
| Schuss 3 | 120 °C |
| Schuss 4 | 135 °C |
| Schuss 5 | 120 °C |
| Düse | 100 °C |

Es wurde ein transparentes, röntgenamorphes in Wasser dispergierbares Granulat erhalten.

## Patentansprüche

1. Verfahren zur Herstellung von festen Dosierungsformen durch Vermischen von mindestens einem polymeren Bindemittel, mindestens einem Wirkstoff und gegebenenfalls üblichen Additiven unter Bildung eines plastischen Gemisches und Formgebung, dadurch gekennzeichnet, dass man als polymeres Bindemittel ein Homo- oder Copolymer von N-Vinylcaprolactam verwendet.

2. Verfahren nach Anspruch 1, dadurch gekennzeichnet, dass man als polymeres Bindemittel ein Homopolymerisat des N-Vinylcaprolactams verwendet.

3. Verfahren nach Anspruch 1, dadurch gekennzeichnet, dass man als polymeres Bindemittel ein Copolymer des N-Vinylcaprolactams verwendet, das mindestens 10 Gew.-%, insbesondere mindestens 25 Gew.-% und besonders bevorzugt mindestens 50 Gew.-% N-Vinylcaprolactam-Einheiten einpolymerisiert enthält.

4. Verfahren nach Anspruch 1 oder 3, dadurch gekennzeichnet, dass man als polymeres Bindemittel ein Copolymer des N-Vinylcaprolactams verwendet, das als Comonomer mindestens ein copolymerisierbares Monomer einpolymerisiert enthält, das ausgewählt ist unter monoethylenisch ungesättigten Mono- und Dicarbonsäuren mit 3 bis 30 Kohlenstoffatomen; Estern und Halbestern dieser Carbonsäuren mit C₁-C₂₄-Alkanolen, C₂-C₄-Alkan-diolen und Mono- oder Di-C₁-C₄-alkylamino-C₂-C₄-alkanolen; Amiden, Mono- und Di-C₁-C₄-alkylamiden und Nitrilen dieser Carbonsäuren, Vinylestern von aliphatischen C₁-C₁₈-Carbonsäu-ren sowie N-Vinylpyrrolidon und den Salzen davon.

5. Verfahren nach Anspruch 4, dadurch gekennzeichnet, dass das Comonomer ausgewählt ist unter monoethylenisch ungesättigten C₃-C₆-Mono- und Dicarbonsäuren und den Estern dieser Carbonsäuren mit C₁-C₁₈-Alkanolen und C₂-C₄-Alkandiolen.

6. Verfahren nach Anspruch 5, dadurch gekennzeichnet, dass das Comonomer ausgewählt ist unter Acrylsäure, Methacrylsäure, Maleinsäure, Estern der Acrylsäure oder Methacrylsäure mit Methanol, Ethanol, n-Butanol, 2-Ethylhexanol, Ethylenglykol und Propylenglykol und insbesondere N-Vinylpyrrolidon.

7. Verfahren nach einem der vorhergehenden Ansprüche, dadurch gekennzeichnet, dass das polymere Bindemittel einen K-Wert im Bereich von 10 bis 100 aufweist.

8. Verfahren nach einem der vorhergehenden Ansprüche, dadurch gekennzeichnet, dass die Herstellung des plastischen Gemisches durch Vermischen und/oder Aufschmelzen der Komponenten in einem Extruder erfolgt.

9. Verfahren nach einem der vorhergehenden Ansprüche zur Herstellung von pharmazeutischen Dosierungsformen, Pflanzenbehandlungsmitteln, Futtermittelzusatzstoffen und -zusätzen, Nahrungsmittelzusätzen und Dosierungsformen für Riechstoffe und Parfümöle.

10. Feste Dosierungsform, erhältlich nach einem Verfahren gemäß einem der Ansprüche 1 bis 9.
